Europäisches Patentamt

European Patent Office

Office européen des brevets

(19)

(11) **EP 1 399 415 B1**

(12) **FASCICULE DE BREVET EUROPEEN**

(45) Date de publication et mention
de la délivrance du brevet:
**16.03.2005 Bulletin 2005/11**

(21) Numéro de dépôt: **02776508.0**

(22) Date de dépôt: **22.05.2002**

(51) Int Cl.7: **C07C 249/04**, C01B 21/14

(86) Numéro de dépôt international:
**PCT/EP2002/005605**

(87) Numéro de publication internationale:
**WO 2002/098842 (12.12.2002 Gazette 2002/50)**

(54) **PROCEDE DE FABRICATION DE L'OXIME DE LA 2,2,4,4-T TRAM THYL-3-PENTANONE ET DE SELS D'HYDROXYLAMMONIUM**

**VERFAHREN ZUR HERSTELLUNG VON 2,2,4,4 TETRAMETHYL-3-PENTANONOXIM UND HYDROXYLAMMONIUMSALZEN**

**METHOD FOR MAKING 2,2,4,4-TETRAMETHYL-3-PENTANONE OXIME AND HYDROXYLAMMONIUM SALTS**

(84) Etats contractants désignés:
**AT BE CH CY DE DK ES FI FR GB GR IE IT LI LU MC NL PT SE TR**

(30) Priorité: **01.06.2001 FR 0107196**

(43) Date de publication de la demande:
**24.03.2004 Bulletin 2004/13**

(73) Titulaire: **Fluorotech, LLC**
**Gainesville, FL 32608 (US)**

(72) Inventeur: **SCHIRMANN, Jean-Pierre**
**F-69600 Oullins (FR)**

(74) Mandataire: **Kaplan, Jean-Pierre**
**10, route de Chalon**
**01340 Montrevel en Bresse (FR)**

(56) Documents cités:
**EP-A- 0 074 472      EP-A- 0 208 311**
**EP-A- 0 267 362      US-A- 3 256 331**

## Description

La présente invention concerne un procédé de fabrication de l'oxime de la 2,2,4,4-tétraméthyl-3-pentanone et de sels d'hydroxylammonium par hydrolyse acide de cette oxime.

Les sels d'hydroxylammonium sont fabriqués à très grande échelle et utilisés presque exclusivement pour produire l'oxime de la cyclohexanone qui est elle-même transformée en caprolactame, monomère du Polyamide 6. Ces sels ont trouvé d'autres applications dans d'autres domaines tels que la désoxygénation des eaux de chaudières, la génération de gaz dans les airbags ou encore plus simplement la génération de l'hydroxylamine base.

Le procédé le plus ancien de fabrication de sels d'hydroxylammonium est le procédé Raschig qui est basé sur une réduction du nitrite d'ammonium par le dioxyde de soufre. Ce procédé n'est plus économique et de plus coproduit des quantités importantes de sulfate d'ammonium difficilement valorisable.

A ce jour, le procédé dominant de fabrication de sels d'hydroxylammonium consiste à réduire l'oxyde nitrique NO par l'hydrogène en présence d'un catalyseur au platine et d'un acide minéral dilué, tel que l'acide chlorhydrique ou l'acide sulfurique, comme décrit dans le brevet US 3313595. L'oxyde nitrique est lui-même obtenu par combustion ménagée de l'ammoniac par l'oxygène moléculaire sur un catalyseur à base de platine, tel qu'un alliage platine-rhodium comme cela est décrit dans le brevet US 3110563. Le rendement global sur l'ammoniac est de l'ordre de 75% sur l'ensemble des deux réactions, qui peut se schématiser par l'équation :

$$NH_3 + 1,5\ H_2 + 1,25\ O_2 + HX \rightarrow NH_2OH,\ HX + 1,5\ H_2O$$

Ce type de procédé nécessite trois matières premières ( l'ammoniac, l'hydrogène et de l'oxygène moléculaire ), des catalyseurs coûteux et facilement empoisonnés et enfin coproduit aussi des oxydes d'azote supérieurs qu'il faut soit valoriser soit détruire.

Il existe aussi un procédé appelé procédé HPO ( Hydroxylamine- Phosphate-Oxime) qui consiste à réduire une solution de nitrate d'ammonium par l'hydrogène en présence d'un catalyseur au palladium et d'acide phosphorique. Il se forme intermédiairement une solution de phosphate d'hydroxylammonium qui est utilisée directement sans isoler le sel pour la fabrication de l'oxime de la cyclohexanone, comme cela est décrit par exemple dans Hydrocarbon Process 51, pages 92-94 (1971). Le rendement global sur l'ammoniac est de l'ordre de 60%.

$$2\ H_3PO_4 + NH_4NO_3 + 3H_2 \rightarrow NH_2OH,\ H_3PO_4 + NH_4H_2PO_4 + 5H_2O$$

L'obtention de sels d'hydroxylammonium à partir d'oximes de cétones est aussi connue et décrite par exemple dans Organic Syntheses Coll. Vol. I, pages 318-321 (1941), mais n'a jamais fait l'objet de production industrielle. C'est ainsi dans le cas de l'acétone :

$$(CH_3)_2\ C=NOH + H_2O + HCl \rightarrow NH_2OH,\ HCl + (CH_3)_2\ C=O$$

EP74 472 décrit un procédé d'hydrolyse en continu d'une cétoxime.

On a aussi proposé de fabriquer directement l'oxime de la cyclohexanone, sans passer par un sel d'hydroxylammonium, en faisant réagir le peroxyde d'hydrogène avec l'ammoniac et la cyclohexanone en présence d'un catalyseur approprié tel que le tungstate de sodium tel que décrit dans Zhur. Obshch. Khim. 30, 1631 (1960), ou d'autres dérivés plus élaborés de l'acide tungstique tels que ceux revendiqués dans le brevet DE 1245371. Les rendements en oxime sont malheureusement trop bas pour pouvoir envisager un procédé industriel qui de plus nécessiterait de résoudre d'importants problèmes techniques de recyclage et de retraitement du catalyseur.

On a proposé plus récemment d'améliorer ce type de procédé, en activant le peroxyde d'hydrogène à l'aide d'une zéolithe adéquat et en particulier d'une titanosilicalite comme cela est décrit par exemple dans le brevet US 4745221. Ce procédé amélioré quant au rendement sur le peroxyde d'hydrogène ne permet cependant pas d'empêcher la formation de nombreux sous-produits de la cyclohexanone en milieu ammoniacal et de ce fait n'a jamais vu le jour.

US 3256331 décrit un procédé de fabrication de l'oxime de la 2,2,4,4-tétraméthyl-3-pentanone.

En poursuivant ses recherches dans le domaine de l'oxydation de l'ammoniac par le peroxyde d'hydrogène, le demandeur a découvert que l'on pouvait obtenir des sels d'hydroxylammonium avec de bons rendements selon l'équation stoechiométrique :

$$NH_3 + H_2O_2 + HX \rightarrow NH_2OH,\ HX + H_2O$$

HX étant un acide, de préférence un acide minéral fort.

Le procédé selon l'invention se subdivise en deux procédés successifs.

Dans un premier stade, l'invention consiste en un procédé de fabrication de l'oxime de la 2,2,4,4-tétraméthyl-3-pentanone, caractérisé en ce qu'on forme un milieu réactionnel contenant de la 2,2,4,4-tétraméthyl-3-pentanone, du peroxyde d'hydrogène, de l'ammoniac et un catalyseur de structure zéolithique et on laisse réagir pour obtenir ladite oxime.

Dans un deuxième stade, on réalise un procédé de fabrication d'un sel d'hydroxylammonium, caractérisé en ce que dans une étape ultérieure au procédé précédent, on sépare du milieu réactionnel l'oxime de la 2,2,4,4-tétraméthyl-3-pentanone que l'on hydrolyse ensuite par de l'eau en présence d'un acide pour donner, d'une part, le sel d'hydroxylammonium de cet acide et d'autre part, de la 2,2,4,4-tétraméthyl-3-pentanone.

Les réactions impliquées sont les suivantes :

$$((CH_3)_3C)_2C=O + H_2O_2 + NH_3 \rightarrow ((CH_3)_3C)_2C=NOH + 2\ H_2O \qquad (1)$$

$$((CH_3)_3C)_2C=NOH + H_2O + HX \rightarrow ((CH_3)_3C)_2C=O + NH_2OH,HX \qquad (2)$$

La 2,2,4,4-tétraméthyl-3-pentanone produite dans la réaction (2) peut être recyclée à la réaction (1).

Une variante du procédé selon la présente invention consiste à utiliser le produit d'autoxydation par l'oxygène ou l'air de l'alcool secondaire correspondant à la 2,2,4,4-tétraméthyl-3-pentanone, c'est-à-dire le di-terbutylcarbinol ou 2,2,4,4-tétraméthyl-3-pentanol selon le schéma réactionnel (3) :

$$((CH_3)_3C)_2CHOH + O_2 \rightarrow ((CH_3)_3C)_2C=O+H_2O_2 \qquad (3)$$

Ainsi, avantageusement, le peroxyde d'hydrogène et la 2,2,4,4-tétraméthyl-3-pentanone sont introduits ou formés dans le milieu réactionnel en tant que produits résultant de l'autoxydation par de l'air ou de l'oxygène moléculaire du 2,2,4,4-tétraméthyl-3-pentanol.

La réaction d'oxydation de l'ammoniac par le peroxyde d'hydrogène en présence de 2,2,4,4-tétraméthyl-3-pentanone ou par les produits d'oxydation à l'oxygène ou l'air du 2,2,4,4-tétramethyl-3-pentanol, soit préformés, soit formés in situ, peut être conduite en discontinu ou en continu mais on préfère opérer selon un processus continu pour des raisons pratiques et économiques. Dans les deux cas, le matériau du ou des réacteurs nécessaires à la mise en oeuvre du procédé doit être compatible avec la stabilité du peroxyde d'hydrogène.

Avantageusement, le milieu réactionnel comporte trois phases : une phase solide (le catalyseur), une phase aqueuse contenant le peroxyde d'hydrogène et l'ammoniac et une phase organique contenant la 2,2,4,4-tétraméthyl-3-pentanone et/ou l'oxime.

L'eau est de loin le meilleur solvant pour solubiliser l'ammoniac, permettant ainsi le bon déroulement de la réaction. La quantité d'eau mise en oeuvre est en général sensiblement égale en poids à la quantité de cétone mise en oeuvre.

On peut aussi, dans le cadre de la présente invention, travailler avec une seule phase liquide en utilisant un tiers solvant permettant de rendre miscible la phase aqueuse avec la phase organique. A cet effet, on préfère les alcools primaires de bas poids moléculaires tels le méthanol ou l'éthanol. Dans ces conditions le milieu réactionnel comporte deux phases : une phase solide (le catalyseur) et une phase liquide résultant de la miscibilité d'une phase aqueuse et d'une phase organique par l'ajout d'un tiers solvant notamment un alcool primaire de bas poids moléculaire.

Avantageusement, le rapport molaire peroxyde d'hydrogène/2,2,4,4-tétraméthyl-3-pentanone est de 0,5 à 2,5 et de préférence de 1 à 1,2.

Avantageusement, le rapport molaire ammoniac/2,2,4,4-tétraméthyl-3-pentanone est de 1 à 5 et de préférence de 1,5 à 2.

En général, le milieu réactionnel est porté à une température allant de 25°C à 100°C et de préférence de 50°C à 80°C.

Avantageusement, le milieu réactionnel est à une pression allant de 1 à 10 bars et de préférence de 1 à 2 bars.

Les catalyseurs utiles dans le cadre de cette invention ont une structure zéolithique par exemple les zéolithes du type Y, les zéolithes ZSM 5, les zéolithes du type ZSM 11 ou encore les zéolithes ZSM décrites dans les brevets EP 129239, EP 141514 et 143642 ou bien encore les zéolithes MB 28 décrites dans le brevet EP21445. On préfère cependant les zéolithes de structure silicalite et plus particulièrement les titanosilicalites telles que celles décrites dans les brevets GB 2024790 et GB 2071071. On utilise en général de 0,1 à 100 g en poids de catalyseur pour 100 g de cétone mise en oeuvre mais on préfère travailler en continu avec un débit de 100 g/h de cétone par gramme de catalyseur.

Lorsque la réaction est terminée, l'oxime de la 2,2,4,4-tétraméthyl-3-pentanone peut être séparée par différentes méthodes connues. On peut par exemple, après séparation du catalyseur solide, procéder à une extraction à l'aide d'un solvant organique non miscible à l'eau tel que le toluène. L'oxime peut être isolée pour être utilisée à l'état pur mais on préfère la transformer directement en sel d'hydroxylammonium par action d'un acide minéral dilué. Les acides préférés sont les acides chlorhydrique, sulfurique et nitrique.

L'hydroxylamine peut être à son tour obtenue par déplacement à partir de l'un de ses sels obtenus par le procédé précédemment décrit, selon des méthodes connues de l'homme de l'art. Par exemple on peut opérer par neutralisation d'une solution de chlorure d'hydroxylammonium à l'aide d'une solution méthanolique de méthylate de sodium. Après filtration du chlorure de sodium sous produit, on dispose d'une solution méthanolique d'hydroxylamine prête à l'emploi.

Le procédé selon la présente invention est particulièrement avantageux, tant sur le plan économique que sur le plan environnemental, en ce qu'il est caractérisé par des réactions propres mettant en jeu le minimum de réactifs, en utilisant le parcours le plus court possible autorisé par la thermodynamique et que de plus les rendements sont très élevés.

L'exemple suivant illustre de façon non limitative la portée de la présente invention :

Exemple : Dans un réacteur équipé d'une agitation, muni d'une double enveloppe alimentée par de l'eau chaude et maintenue à une température de 60°C et inerté par de l'azote, on place successivement 1,5 g de titanosilicalite sous forme de poudre finement divisée et contenant 3,85% en poids de dioxyde de titane, puis 50 ml d'une solution aqueuse d'ammoniac à 30%. Sous agitation, on ajoute alors 14 g de 2,2,4,4-tétraméthyl-3-pentanone (0,1 mole). Le mélange triphasique est homogénéisé sous forte agitation. On attend que la température se stabilise à 60°C et on démarre l'addition goutte à goutte de 11,2 g de peroxyde d'hydrogène à 30% ( 0,1 mole) pendant une durée de une demi-heure. On laisse la réaction se poursuivre encore pendant deux heures à 60°C tout en faisant passer un léger courant d'ammoniac gazeux dans le mélange réactionnel. On refroidit ensuite celui-ci, puis on ajoute 50 ml de toluène et on agite durant quelques minutes. La phase solide est séparée du milieu liquide par filtration. Les deux phases liquides sont à leur tour séparées par décantation, puis la phase aqueuse est extraite par deux fois avec 30 ml de toluène. Les phases toluèniques sont réunies et la solution finale ainsi obtenue pèse 125 g. Une part de 12,5 g de cette solution est soumise à évaporation pour isoler l'oxime de la 2,2,4,4-tetramethyl-3-pentanone et la caractériser après recristallisation dans l'éther de pétrole ( Skellysolve B, bp 60-71°C) par comparaison avec un échantillon d'oxime pure du commerce ( Sigma Aldrich Chimie). On observe un point de fusion de 158°C pour les deux échantillons et des spectres infra-rouges identiques. Le reste de la solution toluènique est traité par 30 ml d'acide chlorhydrique concentré. Après séparation du toluène par décantation, on évapore la phase aqueuse acide à sec sous pression réduite. On isole ainsi 5,9 g de $NH_2OH,HCl$ (point de fusion 151°C après recristallisation dans l'eau ), ce qui correspond à un rendement global de 94% par rapport à $H_2O_2$ et $NH_3$.

### Revendications

**1.** Procédé de fabrication de l'oxime de la 2,2,4,4-tétraméthyl-3-pentanone, **caractérisé en ce qu'**on forme un milieu réactionnel contenant de la 2,2,4,4-tétraméthyl-3-pentanone, du peroxyde d'hydrogène, de l'ammoniac et un catalyseur de structure zéolithique et on laisse réagir pour obtenir ladite oxime.

**2.** Procédé suivant la revendication 1, **caractérisé en ce que** le peroxyde d'hydrogène et la 2,2,4,4-tétraméthyl-3-pentanone sont introduits ou formés dans le milieu réactionnel en tant que produits résultant de l'autoxydation par de l'air ou de l'oxygène moléculaire du 2,2,4,4-tétraméthyl-3-pentanol.

**3.** Procédé suivant la revendication 1 ou 2, **caractérisé en ce que** le milieu réactionnel comporte trois phases : une phase solide (le catalyseur), une phase aqueuse contenant le peroxyde d'hydrogène et l'ammoniac et une phase organique contenant la 2,2,4,4-tétraméthyl-3-pentanone et/ou l'oxime.

**4.** Procédé suivant la revendication 1 ou 2, **caractérisé en ce que** le milieu réactionnel comporte deux phases : une phase solide (le catalyseur) et une phase liquide résultant de la miscibilité d'une phase aqueuse et d'une phase organique par l'ajout d'un tiers solvant notamment un alcool primaire de bas poids moléculaire.

**5.** Procédé suivant l'une des revendications 1 à 4, **caractérisé en ce que** le rapport molaire peroxyde d'hydrogène/ 2,2,4,4-tétraméthyl-3-pentanone est de 0,5 à 2,5 et de préférence de 1 à 1,2.

6. Procédé suivant l'une des revendications 1 à 5, **caractérisé en ce que** le rapport molaire ammoniac/ 2,2,4,4-tétraméthyl-3-pentanone est de 1 à 5 et de préférence de 1,5 à 2.

7. Procédé suivant l'une des revendications 1 à 6, **caractérisé en ce que** le milieu réactionnel est porté à une température allant de 25°C à 100°C et de préférence de 50°C à 80°C.

8. Procédé suivant l'une des revendications 1 à 7, **caractérisé en ce que** le milieu réactionnel est à une pression allant de 1 à 10 bars et de préférence de 1 à 2 bars.

9. Procédé suivant l'une des revendications 1 à 8, **caractérisé en ce que** le catalyseur de structure zéolithique est choisi parmi les zéolithes de structure silicalite notamment les titanosilicalites.

10. Procédé de fabrication d'un sel d'hydroxylammonium, **caractérisé en ce que** dans une étape ultérieure au procédé suivant l'une des revendications 1 à 9, on sépare du milieu réactionnel l'oxime de la 2,2,4,4-tétraméthyl-3-pentanone que l'on hydrolyse ensuite par de l'eau en présence d'un acide pour donner, d'une part, le sel d'hydroxylammonium de cet acide et d'autre part, de la 2,2,4,4-tétraméthyl-3-pentanone.

## Claims

1. A process for the manufacture of 2,2,4,4-tetramethyl-3-pentanone oxime, **characterized in that** a reaction medium comprising 2,2,4,4-tetramethyl-3-pentanone, hydrogen peroxide, ammonia and a catalyst with a zeolite structure is formed and reaction is allowed to take place to produce said oxime.

2. The process as claimed in claim 1, **characterized in that** the hydrogen peroxide and the 2,2,4,4-tetramethyl-3-pentanone are introduced into or formed in the reaction medium as products resulting from the autoxidation by air or molecular oxygen of 2,2,4,4-tetramethyl-3-pentanol.

3. The process as claimed in claim 1 or 2, **characterized in that** the reaction medium comprises three phases: a solid phase (the catalyst), an aqueous phase, comprising the hydrogen peroxide and the ammonia, and an organic phase, comprising the 2,2,4,4-tetramethyl-3-pentanone and/or the oxime.

4. The process as claimed in claim 1 or 2, **characterized in that** the reaction medium comprises two phases: a solid phase (the catalyst) and a liquid phase resulting from the miscibility of an aqueous phase and of an organic phase by the addition of a third solvent, in particular a primary alcohol of low molecular weight.

5. The process as claimed in one of claims 1 to 4, **characterized in that** the hydrogen peroxide/2,2,4,4-tetramethyl-3-pentanone molar ratio is from 0.5 to 2.5 and preferably from 1 to 1.2.

6. The process as claimed in one of claims 1 to 5, **characterized in that** the ammonia/2,2,4,4-tetramethyl-3-pentanone molar ratio is from 1 to 5 and preferably from 1.5 to 2.

7. The process as claimed in one of claims 1 to 6, **characterized in that** the reaction medium is brought to a temperature ranging from 25°C to 100°C and preferably from 50°C to 80°C.

8. The process as claimed in one of claims 1 to 7, **characterized in that** the reaction medium is at a pressure ranging from 1 to 10 bar and preferably from 1 to 2 bar.

9. The process as claimed in one of claims 1 to 8, **characterized in that** the catalyst with a zeolite structure is chosen from zeolites with a silicalite structure, in particular titanium silicalites.

10. A process for the manufacture of a hydroxylammonium salt, **characterized in that**, in a stage subsequent to the process as claimed in one of claims 1 to 9, the 2,2,4,4-tetramethyl-3-pentanone oxime is separated from the reaction medium and is subsequently hydrolyzed by water in the presence of an acid to give, on the one hand, the hydroxylammonium salt of this acid and, on the other hand, 2,2,4,4-tetramethyl-3-pentanone.

**Patentansprüche**

1. Verfahren zur Herstellung von 2,2,4,4-Tetramethyl-3-pentanonoxim, **dadurch gekennzeichnet, daß** man ein 2,2,4,4-Tetramethyl-3-pentanon, Wasserstoffperoxid, Ammoniak und einen Katalysator mit Zeolithstruktur enthaltendes Reaktionsmedium herstellt und die Reaktion zum Oxim ablaufen läßt.

2. Verfahren nach Anspruch 1, **dadurch gekennzeichnet, daß** man das Wasserstoffperoxid und das 2,2,4,4-Tetramethyl-3-pentanon als Produkte der Autoxidation von 2,2,4,4-Tetramethyl-3-pentanol mit Luft oder molekularem Sauerstoff in das Reaktionsmedium einträgt oder darin bildet.

3. Verfahren nach Anspruch 1 oder 2, **dadurch gekennzeichnet, daß** das Reaktionsmedium drei Phasen enthält: eine feste Phase (den Katalysator), eine wäßrige Phase, die das Wasserstoffperoxid und das Ammoniak enthält, und eine organische Phase, die das 2,2,4,4-Tetramethyl-3-pentanon und/oder das Oxim enthält.

4. Verfahren nach Anspruch 1 oder 2, **dadurch gekennzeichnet, daß** das Reaktionsmedium zwei Phasen enthält: eine feste Phase (den Katalysator) und eine flüssige Phase, die sich aus der Mischbarkeit einer wäßrigen Phase und einer organischen Phase durch Zusatz eines dritten Lösungsmittels, insbesondere eines primären Alkohols mit niedrigem Molekulargewicht, ergibt.

5. Verfahren nach einem der Ansprüche 1 bis 4, **dadurch gekennzeichnet, daß** das Molverhältnis von Wasserstoffperoxid zu 2,2,4,4-Tetramethyl-3-pentanon 0,5 bis 2,5 und vorzugsweise 1 bis 1,2 beträgt.

6. Verfahren nach einem der Ansprüche 1 bis 5, **dadurch gekennzeichnet, daß** das Molverhältnis von Ammoniak zu 2,2,4,4-Tetramethyl-3-pentanon 1 bis 5 und vorzugsweise 1,5 bis 2 beträgt.

7. Verfahren nach einem der Ansprüche 1 bis 6, **dadurch gekennzeichnet, daß** man das Reaktionsmedium auf eine Temperatur im Bereich von 25°C bis 100°C und vorzugsweise von 50°C bis 80°C bringt.

8. Verfahren nach einem der Ansprüche 1 bis 7, **dadurch gekennzeichnet, daß** das Reaktionsmedium unter einem Druck im Bereich von 1 bis 10 bar und vorzugsweise von 1 bis 2 bar steht.

9. Verfahren nach einem der Ansprüche 1 bis 8, **dadurch gekennzeichnet, daß** man den Katalysator mit Zeolithstruktur unter Zeolithen mit Silicalitstruktur, insbesondere Titansilicaliten, auswählt.

10. Verfahren zur Herstellung eines Hydroxylammoniumsalzes, **dadurch gekennzeichnet, daß** man in einem dem Verfahren nach einem der Ansprüche 1 bis 9 nachgeschalteten Schritt das 2,2,4,4-Tetramethyl-3-pentanonoxim aus dem Reaktionsmedium abtrennt und dann in Gegenwart einer Säure mit Wasser hydrolysiert, wobei man zum einen das Hydroxylammoniumsalz dieser Säure und zum anderen 2,2,4,4-Tetramethyl-3-pentanon erhält.